# EUROPEAN PATENT SPECIFICATION

(11) **EP 3 421 039 B1**
(45) Date of publication and mention of the grant of the patent: **06.10.2021**
(21) Application number: 17756670.0
(22) Date of filing: 24.02.2017
(51) Int. Cl.: A61K 31/4709, A61K 39/395, A61K 45/00, A61P 35/00, A61P 43/00, C07K 16/18

(54) **DRUG FOR CANCER THERAPY CHARACTERIZED BY ADMINISTERING COMBINATION BETWEEN AXL INHIBITOR AND IMMUNE CHECKPOINT INHIBITOR**
MEDIKAMENT ZUR BEHANDLUNG VON KREBS DURCH VERABREICHUNG EINER KOMBINATION AUS EINEM AXL-HEMMER UND IMMUN-CHECKPOINT-INHIBITOR
MÉDICAMENT POUR LE TRAITEMENT DU CANCER CARACTÉRISÉ PAR L'ADMINISTRATION D'UNE ASSOCIATION D'UN INHIBITEUR DE L'AXL ET D'UN INHIBITEUR DE POINT DE CONTRÔLE IMMUNITAIRE

(30) Priority: 26.02.2016 JP 2016035206
(43) Date of publication of application: 02.01.2019
(73) Proprietor: ONO Pharmaceutical Co., Ltd., Osaka-shi, Osaka 541-8526 (JP)
(72) Inventor: TANAKA, Kohei, Mishima-gun Osaka 618-8585 (JP); YASUHIRO, Tomoko, Mishima-gun Osaka 618-8585 (JP)
(74) Representative: Grünecker Patent- und Rechtsanwälte PartG mbB
(86) International application number: PCT/JP2017/007219
(87) International publication number: WO 2017/146236

(56) References cited:
- WO-A1-2015/012298
- WO-A1-2015/012298
- WO-A1-2016/193680
- Gausdal: "Abstract B014: BGB324, a selective small molecule inhibitor of the receptor tyrosine kinase AXL, enhances immune checkpoint inhibitor efficacy | Cancer Immunology Research", Cancer Immunol Res 2016;4(1 Suppl):Abstract nr B014., 1 January 2016 (2016-01-01), XP55519427, Retrieved from the Internet: URL:http://cancerimmunolres.aacrjournals.o rg/content/4/1_Supplement/B014 [retrieved on 2018-10-26]
- P. A. OTT ET AL: "CTLA-4 and PD-1/PD-L1 Blockade: New Immunotherapeutic Modalities with Durable Clinical Benefit in Melanoma Patients", CLINICAL CANCER RESEARCH, vol. 19, no. 19, 1 October 2013 (2013-10-01), pages 5300-5309, XP55458362, US ISSN: 1078-0432, DOI: 10.1158/1078-0432.CCR-13-0143
- GRO GAUSDAL et al.: "Abstract B014: BGB324, a selective small molecule inhibitor of the receptor tyrosine kinase AXL, enhances immune checkpoint inhibitor efficacy", Cancer Immunology Research, vol. 4, no. Suppl 1 January 2016 (2016-01), XP055519427, Retrieved from the Internet: URL:http://cancerimmunolres.aacrjournals.o rg/content/4/1_Supplement/B014 [retrieved on 2017-03-22]
- KUNIKO SUNAMI: "CTLA-4 o Hyoteki to shita Gan Men'eki Ryoho", Japanese Journal of Clinical Medicine (special extra issue) Saishin Gan Yakubutsu Ryohogaku, vol. 72, 20 February 2014 (2014-02-20), pages 280-285,
- SHIN FOONG NIGLOW et al.: "Anti-TIM3 Antibody Promotes T Cell IFN-y-Mediated Antitumor Immunity and Suppresses Established Tumors", Cancer Research, vol. 71, no. 10, 15 May 2011 (2011-05-15), pages 3540-3551, XP055181433,
- PASERO CHRISTINE et al.: "Interfering with coinhibitory molecules: BTLA/HVEM as new targets to enhance anti-tumor immunity", Immunology Letters, vol. 151, no. 1-2, March 2013 (2013-03), pages 71-75, XP055251710,
- YOSHIZAWA TOSHIO et al.: "Abstract LB-218: Development of Axl/Mer inhibitor , ONO-9330547: preclinical evidence supporting the combination with immunotherapeutics", Cancer Research, vol. 76, no. 14 S July 2016 (2016-07), XP055537320, Retrieved from the Internet: URL:http://cancerres.aacrjournals.org/cont ent/76/14 _ Supplement/LB-218 [retrieved on 2017-03-22]

### Sequence Listing

<110> ONO PHARMACEUTICAL CO., LTD.
<120> A medicament comprising a combination of an Axl inhibitor and an
   immune checkpoint inhibitor for cancer treatment
<130> P160810WO
<150> JP2016-035206
   <151> 2016-02-26
<160> 8
<210> 1
   <211> 113
   <212> PRT
   <213> Homo sapiens
<400> 1
<210> 2
   <211> 107
   <212> PRT
   <213> Homo sapiens
<400> 2
<210> 3
   <211> 5
   <212> PRT
   <213> Homo sapiens
<400> 3
   Asn Ser Gly Met His
   1               5
<210> 4
   <211> 17
   <212> PRT
   <213> Homo sapiens
<400> 4
   Val Ile Trp Tyr Asp Gly Ser Lys Arg Tyr Tyr Ala Asp Ser Val Lys
   1               5                   10                  15
   Gly
<210> 5
   <211> 4
   <212> PRT
   <213> Homo sapiens
<400> 5
   Asn Asp Asp Tyr
   1
<210> 6
   <211> 11
   <212> **PRT**
   <213> Homo sapiens
<400> 6
   Arg Ala Ser Gln Ser Val Ser Ser Tyr Leu Ala
   1               5                   10
<210> 7
   <211> 7
   <212> PRT
   <213> Homo sapiens
<400> 7
   Asp Ala Ser Asn Arg Ala Thr
   1               5
<210> 8
   <211> 9
   <212> PRT
   <213> Homo sapiens
<400> 8
   Gln Gln Ser Ser Asn Trp Pro Arg Thr
   1               5

## Description

### [Technical Field]

The present invention, relates to a drug for cancer therapy, obtained by combination of an Axl inhibitor and an immune checkpoint inhibitor, wherein the Axl inhibitor is N-{5-[(6,7-dimethoxy-4-quinolinyl)oxy]-2-pyridinyl}-2,5-dioxo-1-phenyl-1,2,5,6,7,8-hexahydro-3-quinolinecarboxamide, a salt thereof, a solvate thereof, an N-oxide thereof, or a prodrug thereof.

### [Background Art]

Cancer cells and cancer microenvironment include various immune checkpoint molecules that inhibit immune responses against cancer. An immune checkpoint inhibitor is a new therapy that releases the immunosuppressive mechanism and activates immune responses against cancer. As immune checkpoint inhibitors, ipilimumab as an anti-CTLA-4 (cytotoxic T lymphocyte-associated antigen-4) antibody and nivolumab and pembrolizumab as an anti-PD-1 (programmed cell death-1) antibody have already been approved at home and abroad, and have been used for cancer therapy.

Axl (also known as: UFO, ARK, Tyro7) is a receptor tyrosine kinase belonging to a TAM family (Axl, Mer and Tyro3) cloned from tumor cells. Gas6 (growth-arrest-specific protein 6) cloned as a gene specifically expressed at the time of cell proliferation arrest is known as a ligand for Axl. Axl activated by binding of Gas6 transfers a signal via phosphorylation. Since the signal activates an Erkl/2 pathway or a PI3K/Akt pathway, the activation of Axl is known to be involved in pathologic conditions of cancer, immune system diseases, circulatory system diseases, and the like (see NPL 1).

In particular, the relation between Axl and various types of cancers is well known. For example, it is known that the expression of Axl is involved in metastasis and prognosis of breast cancer (see NPL 2), and that Axl is involved in the pathologic conditions of acute myeloid leukemia (AML) (see NPL 3). Furthermore, it is reported that a TAM family including Axl is expressed in immunocytes such as dendritic cells or macrophages, and suppressively regulates anti-tumor immunization (see NPL 4). Therefore, it is considered that compounds which inhibit the activation of Axl are useful for treatment of various types of cancers, immune system diseases, and circulatory system diseases. NPL 5 discloses a small-molecule substance termed BGB324 that inhibits Axl signaling and reports that AXL is an important regulator of tumor placicity; NPL 5 evaluated whether BGB324 enhances the effect of immune checkpoint blockade in syngeneic cancer mouse models. NPL 6 reviews the impact of CTLA-4 and PD-1/PD-L1 (immune checkpoint) blockade with monoclonal antibodies in melanoma patients.

PTL 1 discloses that a compound represented by the general formula (I) has Axl inhibitory action, and is useful as a therapeutic agent for cancer (see PTL 1).

PTL 2 discloses that further combining an Axl inhibitor with the combination of an anti-CTLA-4 antibody and an anti-PD-1 antibody or the combination of an anti-PD-1 antibody and an anti-PD-Ll (programmed cell death-ligand 1) antibody is useful for cancer therapy (see PTL 2).

### [Citation List]

### [Patent Literature]

[PTL 1] WO2015/012298
[PTL 2] WO2016/193680

### [Non Patent Literature]

[NPL 1] Clinical Science, Vol. 122, pp. 361-368, 2012
[NPL 2] Proceedings of the national academy of sciences of the United States of America, Vol. 107, No. 3, pp. 1124-1129, 2010
[NPL 3] Blood, Vol. 121, pp. 2064-2073, 2013
[NPL 4] Nature Reviews Cancer, Vol. 14, pp. 769-785, 2014
[NPL 5] Gausdal, et al., Cancer Immunol. Res. 4, (2016), Abstract B014, January 2016
[NPL 6] Ott et al., Clin. Cancer Res. 19(19, Oct. 1, 2013), p. 5300-5309

### [Summary of Invention]

### [Technical Problem]

A problem to be solved by the present invention is to find an effective treatment method for cancer and to provide the treatment method as a pharmaceutical preparation.

### [Solution to problem]

In order to solve the above-mentioned problem, the inventors of the present invention have keenly studied, and as a result, surprisingly, the inventors have found that a combination of an Axl inhibitor and an immune checkpoint inhibitor (hereinafter, also referred to as a "combination of the present invention"), wherein the Axl inhibitor is N-{5-[(6,7-dimethoxy-4-quinolinyl)oxy]-2-pyridinyl}-2,5-dioxo-1-phenyl-1,2,5,6,7,8-hexahydro-3-quinolinecarboxamide, a salt thereof, a solvate thereof, an N-oxide thereof, or a prodrug thereof, can solve the above-mentioned problems, and have completed the present invention.

In other words, the present invention relates to:
[1] a drug for cancer therapy, obtained by a combination of an Axl inhibitor and an immune checkpoint inhibitor, the Axl inhibitor being N-{5-[(6,7-dimethoxy-4-quinolinyl)oxy]-2-pyridinyl}-2,5-dioxo-1-phenyl-1,2,5,6,7,8-hexahydro-3-quinolinecarboxamide, a salt thereof, a solvate thereof, an N-oxide thereof, or a prodrug thereof,
[2] the drug according to the above-mentioned [1] , wherein the Axl inhibitor is N-{5-[(6,7-dimethoxy-4-quinolinyl)oxy]-2-pyridinyl}-2,5-dioxo-1-phenyl -1,2,5,6,7,8-hexahydro-3-quinolinecarboxamide, a pharmaceutically acceptable salt thereof or a hydrate thereof,
[3] the drug according to any one of the above-mentioned [1] to [2], wherein the immune checkpoint inhibitor is an inhibitor of a PD-1,
[4] the drug according to any one of the above-mentioned [1] to [3], wherein the immune checkpoint inhibitor is an anti-PD-1 antibody (for example, nivolumab (OPDIVO (registered trademark)), REGN-2810, Pembrolizumab (KEYTRUDA (registered trademark)), PDR-001, BGB-A317, AMP-514 (MEDI0680), BCD-100, IBI-308, JS-001, PF-06801591, and TSR-042),
[5] the drug according to any one of the above-mentioned [1] to [4], wherein the cancer is leukemia (for example, acute myeloid leukemia, chronic myeloid leukemia, acute lymphatic leukemia, and chronic lymphatic leukemia), malignant lymphoma (Hodgkin lymphoma and non-Hodgkin lymphoma (for example, adult T-cell leukemia, follicular lymphoma, and diffuse large B-cell lymphoma)), multiple myeloma, osteomyelodysplasia syndrome, head and neck cancer, esophageal cancer, esophageal adenocarcinoma, gastric cancer, colon cancer, colorectal cancer, rectum cancer, liver cancer (for example, hepatocellular cancer), gallbladder cancer and bile duct cancer, biliary tract cancer, pancreatic cancer, thyroid cancer, lung cancer (for example, non-small cell lung cancer (for example, squamous epithelium non-small cell lung cancer, non-squamous epithelium non-small cell lung cancer), and small-cell lung cancer), breast cancer, ovarian cancer (for example, serous ovarian cancer), cervical cancer, uterine body cancer, endometrial cancer, vaginal cancer, vulvar cancer, renal cancer (for example, renal cell carcinoma), urothelial carcinoma (for example, urinary bladder cancer, and upper urinary tract cancer), prostate cancer, testicular tumor (for example, germ cell cancer), bone and soft tissue sarcoma, skin cancer (for example, uveal malignant melanoma, malignant melanoma, and Merkel cell cancer), glioma, brain tumor (for example, glioblastoma), pleural mesothelioma, and unknown primary cancer),
[6] the drug according to any one of the above-mentioned [1] to [5], wherein the cancer is colon cancer or pancreatic cancer,
[7] a drug for cancer therapy, obtained by a combination of N-{5-[(6,7-dimethoxy-4-quinolinyl)oxy]-2-pyridinyl)-2,5-dioxo-1-phenyl-1,2,5,6,7,8-hexahydro-3-quinolinecarboxamide, a pharmaceutically acceptable salt thereof, or a hydrate thereof, and an immune checkpoint inhibitor as defined in [8],
[8] the drug according to the above-mentioned [7], wherein the immune checkpoint inhibitor is an, anti-PD-1 antibody (for example, nivolumab (OPDIVO (registered trademark)), REGN-2810, Pembrolizumab (KEYTRUDA (registered trademark)), PDR-001, BGB-A317, AMP-514 (MEDI0680), BCD-100, IBI-308, JS-001, PF-06801591, and TSR-042),
[9] the drug according to any one of the above-mentioned [7] to [8], wherein the cancer is leukemia (for example, acute myeloid leukemia, chronic myeloid leukemia, acute lymphatic leukemia, and chronic lymphatic leukemia), malignant lymphoma (Hodgkin lymphoma and non-Hodgkin lymphoma (for example, adult T-cell leukemia, follicular lymphoma, and diffuse large B-cell lymphoma)), multiple myeloma, osteomyelodysplasia syndrome, head and neck cancer, esophageal cancer, esophageal adenocarcinoma, gastric cancer, colon cancer, colorectal cancer, rectum cancer, liver cancer (for example, hepatocellular cancer), gallbladder cancer and bile duct cancer, biliary tract cancer, pancreatic cancer, thyroid cancer, lung cancer (for example, non-small cell lung cancer (for example, squamous epithelium non-small cell lung cancer, non-squamous epithelium non-small cell lung cancer), and small-cell lung cancer), breast cancer, ovarian cancer (for example, serous ovarian cancer), cervical cancer, uterine body cancer, endometrial cancer, vaginal cancer, vulvar cancer, renal cancer (for example, renal cell carcinoma), urothelial carcinoma (for example, urinary bladder cancer, and upper urinary tract cancer), prostate cancer, testicular tumor (for example, germ cell cancer), bone and soft tissue sarcoma, skin cancer (for example, uveal malignant melanoma, malignant melanoma, and Merkel cell cancer), glioma, brain tumor (for example, glioblastoma), pleural mesothelioma, and unknown primary cancer),
[10] the drug according to any one of the above-mentioned [7] to [9], wherein the cancer is colon cancer or pancreatic cancer,
[11] means for treating cancer, characterized in that effective amounts of an Axl inhibitor and an immune checkpoint inhibitor, respectively, are administered to a mammalian (preferably, a human patient), wherein the Axl inhibitor is a compound described in the above [1], a salt thereof, a solvate thereof, an N-oxide thereof, or a prodrug thereof,
[12] a combination of an Axl inhibitor and an immune checkpoint inhibitor for use in a method of cancer therapy, wherein the Axl inhibitor is a compound described in the above [1], a salt thereof, a solvate thereof, an N-oxide thereof, or a prodrug thereof,
[13] a drug for use in a method of treating cancer, characterized in that an Axl inhibitor and an immune checkpoint inhibitor are to be administered in combination, the Axl inhibitor being as defined above, a salt thereof, a solvate thereof, an N-oxide thereof, or a prodrug thereof, wherein the immune checkpoint inhibitor is an anti-PD-1 antibody (for example, nivolumab (OPDIVO (registered trademark)), REGN-2810, Pembrolizumab (KEYTRUDA (registered trademark)), PDR-001, BGB-A317, AMP-514 (MEDI0680), BCD-100, IBI-308, JS-001, PF-06801591, and TSR-042)
[14] the drug according to [13], wherein the cancer is leukemia (for example, acute myeloid leukemia, chronic myeloid leukemia, acute lymphatic leukemia, and chronic lymphatic leukemia), malignant lymphoma (Hodgkin lymphoma and non-Hodgkin lymphoma (for example, adult T-cell leukemia, follicular lymphoma, and diffuse large B-cell lymphoma)), multiple myeloma, osteomyelodysplasia syndrome, head and neck cancer, esophageal cancer, esophageal adenocarcinoma, gastric cancer, colon cancer, colorectal cancer, rectum cancer, liver cancer (for example, hepatocellular cancer), gallbladder cancer and bile duct cancer, biliary tract cancer, pancreatic cancer, thyroid cancer, lung cancer (for example, non-small cell lung cancer (for example, squamous epithelium non-small cell lung cancer, non-squamous epithelium non-small cell lung cancer), and small-cell lung cancer), breast cancer, ovarian cancer (for example, serous ovarian cancer), cervical cancer, uterine body cancer, endometrial cancer, vaginal cancer, vulvar cancer, renal cancer (for example, renal cell carcinoma), urothelial carcinoma (for example, urinary bladder cancer, and upper urinary tract cancer), prostate cancer, testicular tumor (for example, germ cell cancer), bone and soft tissue sarcoma, skin cancer (for example, uveal malignant melanoma, malignant melanoma, and Merkel cell cancer), glioma, brain tumor (for example, glioblastoma), pleural mesothelioma, and unknown primary cancer),
[15] the drug according to any one of the above-mentioned [13] to [14], wherein the cancer is colon cancer or pancreatic cancer,
[16] a drug for cancer therapy, characterized in that N-{5-[(6,7-dimethoxy-4-quinolinyl)oxy]-2-pyridinyl}-2,5-dioxo-1-phenyl-1 ,2,5,6,7,8-hexahydro-3-quinolinecarboxamide, a pharmaceutically acceptable salt thereof, or a hydrate thereof are to be administered in combination with an immune checkpoint inhibitor, wherein the immune checkpoint inhibitor is an anti-PD-1 antibody (for example, nivolumab (OPDIVO (registered trademark)), REGN-2810, Pembrolizumab (KEYTRUDA (registered trademark)), PDR-001, BGB-A317, AMP-514 (MEDI0680), BCD-100, IBI-308, JS-001, PF-06801591, and TSR-042),
[17] the drug according to any one of the above-mentioned [16], wherein the cancer is leukemia (for example, acute myeloid leukemia, chronic myeloid leukemia, acute lymphatic leukemia, and chronic lymphatic leukemia), malignant lymphoma (Hodgkin lymphoma and non-Hodgkin lymphoma (for example, adult T-cell leukemia, follicular lymphoma, and diffuse large B-cell lymphoma)), multiple myeloma, osteomyelodysplasia syndrome, head and neck cancer, esophageal cancer, esophageal adenocarcinoma, gastric cancer, colon cancer, colorectal cancer, rectum cancer, liver cancer (for example, hepatocellular cancer), gallbladder cancer and bile duct cancer, biliary tract cancer, pancreatic cancer, thyroid cancer, lung cancer (for example, non-small cell lung cancer (for example, squamous epithelium non-small cell lung cancer, non-squamous epithelium non-small cell lung cancer), and small-cell lung cancer), breast cancer, ovarian cancer (for example, serous ovarian cancer), cervical cancer, uterine body cancer, endometrial cancer, vaginal cancer, vulvar cancer, renal cancer (for example, renal cell carcinoma), urothelial carcinoma (for example, urinary bladder cancer, and upper urinary tract cancer), prostate cancer, testicular tumor (for example, germ cell cancer), bone and soft tissue sarcoma, skin cancer (for example, uveal malignant melanoma, malignant melanoma, and Merkel cell cancer), glioma, brain tumor (for example, glioblastoma), pleural mesothelioma, and unknown primary cancer),
[18] the drug according to any one of the above-mentioned [16] to [17], wherein the cancer is colon cancer or pancreatic cancer,
[19] a therapeutic agent for cancer, including an Axl inhibitor as an active component characterized in being administered in combination with an immune checkpoint inhibitor, wherein the Axl inhibitor is as defined above.
[20] a therapeutic agent for cancer, including an immune checkpoint inhibitor as an active component characterized in being administered in combination with an Axl inhibitor, wherein the Axl inhibitor is as defined above,
[20] a therapeutic agent for cancer, including N-{5-[(6,7-dimethoxy-4-quinolinyl)oxy]-2-pyridinyl}-2,5-dioxo-1-phenyl-1,2,5,6,7,8-hexahydro-3-quinolinecarboxamide, a pharmaceutically acceptable salt thereof and a hydrate thereof as an active component characterized in being administered in combination with an immune checkpoint inhibitor as defined above,
[21] a therapeutic agent for cancer, including an immune checkpoint inhibitor as defined above as an active component characterized in being administered in combination with N-{5-[(6,7-dimethoxy-4-quinolinyl)oxy]-2-pyridinyl}-2,5-dioxo-1-phenyl-1,2,5,6,7,8-hexahydro-3-quinolinecarboxamide, a pharmaceutically acceptable salt thereof, and a hydrate thereof,
[22] the agent according to the above-mentioned [39] or [42], wherein the cancer is as defined above,
[23] use of a combination of an Axl inhibitor and an immune checkpoint inhibitor for manufacturing a drug or therapeutic agent for cancer therapy, wherein the Axl inhibitor is as described in the above [1], a salt thereof, a solvate thereof, an N-oxide thereof, or a prodrug thereof.

### [Advantageous Effects of Invention]

A combination of the present invention is useful for cancer therapy.

### [Brief Description of Drawings]

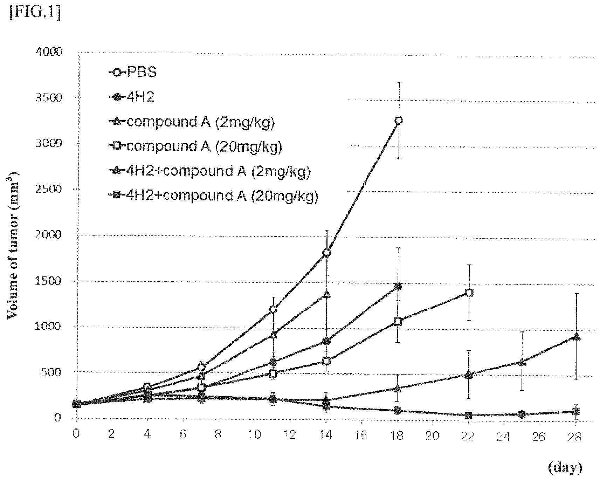

[FIG. 1] FIG. 1 shows an anti-tumor action on a mouse colon cancer cell line MC38 subcutaneous cancer-bearing model by combined use of N-{5-[(6,7-dimethoxy-4-quinolinyl)oxy]-2-pyridinyl}-2,5-dioxo-1-phenyl-1,2,5,6,7,8-hexahydro-3-quinolinecarboxamide (the compound A in the drawing) and an anti-PD-1 antibody: 4H2. The ordinate shows the volume of tumors (mm³), and the abscissa shows days after group assignment (administrations were started on Day 0, respectively). Furthermore, values of each group indicate mean values ± standard errors.

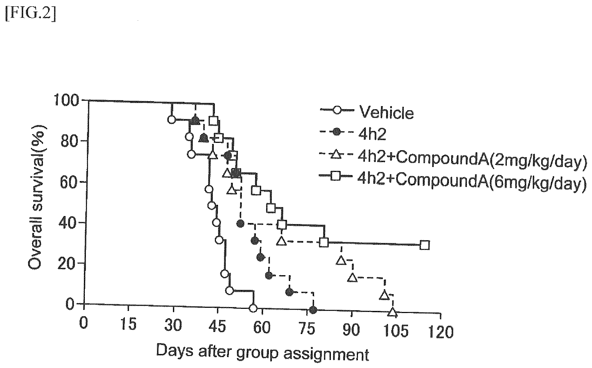

[FIG. 2] FIG. 2 shows an anti-tumor action on a mouse pancreatic cancer cell line Pan02 orthotopic implantation model by combined use of N-{5-[(6,7-dimethoxy-4-quinolinyl)oxy]-2-pyridinyl} -2,5-dioxo-1-phenyl-1,2,5,6,7,8-hexahydro-3-quinolinecarboxamide (compound A in the drawing) and an anti-PD-1 antibody: 4H2. The ordinate shows the survival rate (%), and the abscissa shows days after group assignment (administrations were started on Day 0, respectively).

### [Description of Embodiments]

### (1) Axl inhibitor

In one aspect, an Axl inhibitor to be used for the combination of the present disclosure is a compound represented by the general formula (I) described in WO2015/012298: 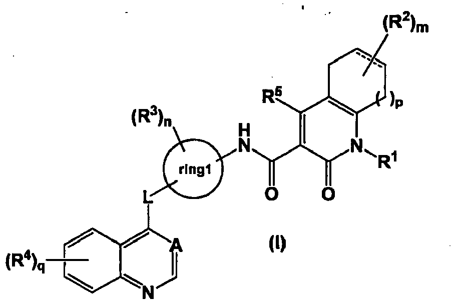
[wherein in the formula, R¹ represents (1) a C1―8 alkyl group optionally substituted with one to five R¹¹, (2) a C3―7 carbon ring optionally substituted with one to five R¹²; or (3) a 4- to 7-membered heterocycle optionally substituted with one to five R^{1 3}; and when the C1―8 alkyl group represented by R¹ is a branched alkyl group, C1―3 alkyl groups branched from the same carbon atom may together form a saturated C3―7 carbon ring;
R² represents (1) a C1―4 alkyl group, (2) a halogen atom, (3) a C1―4 haloalkyl group, (4) an oxo group, or (5) an -OR²¹ group;
R³ represents (1) a C1―4 alkyl group, (2) a halogen atom, or (3) a C1-4 haloalkyl group;
R⁴ represents (1) a C1-4 alkoxy group, (2) a C1―4 haloalkyl group, or (3) an -OR⁴¹ group;
R⁵ represents (1) a hydrogen atom, (2) a C1-4 alkyl group, (3) a halogen atom, (4) a C1-4 haloalkyl group, or (5) an -OR²¹ group;
R¹¹ represents (1) an -OR¹⁰¹ group, (2) an SO₂ R¹⁰² group, (3) an NR¹⁰³ R¹⁰⁴ group, or (4) a C3-7 carbon ring optionally substituted with one to three halogen atoms;
R¹² represents (1) a C1-4 alkyl group, or (2) a halogen atom;
R¹³ represents (1) a C1-4 alkyl group, or (2) a halogen atom;
R²¹ represents (1) a hydrogen atom, or (2) a C1―4 alkyl group;
R⁴¹ represents (1) a hydrogen atom, (2) a C1-8 alkylene group substituted with one to two substituents selected from the group consisting of (a) a 5- to 7-membered cyclic group, (b) NR⁴⁰¹ R⁴⁰², and (c) a hydroxyl group, or (3) a C2-8 alkenylene group substituted with one to two substituents selected from the group consisting of (a) a 5- to 7-membered cyclic group, (b) NR⁴⁰¹ R⁴⁰², and (c) a hydroxyl group;
R¹⁰¹ represents (1) a hydrogen atom, or (2) a C1-4 alkyl group;
R¹⁰² represents (1) a hydrogen atom, or (2) a C1―4 alkyl group;
R¹⁰³ and R¹⁰⁴ each independently represent (1) a hydrogen atom, or (2) a C1―4 alkyl group;
R⁴⁰¹ and R⁴⁰² each independently represent (1) a hydrogen atom, or (2) a C1―4 alkyl group;
A represents (1) CH, or (2) a nitrogen atom;
L represents (1) -O-, (2) -NH-, (3) -C(O)-, (4) -CR⁶R⁷-, (5) -S-, (6) -S(O)-, or (7) - S(O)₂-;
R⁶ and R⁷ each independently represent (1) a hydrogen atom, (2) a halogen atom, (3) a C1―4 alkyl, (4) a hydroxyl group, or (5) NH₂;
ring1 represents a 5- to 7-membered cyclic group;
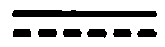 represents a single bond or a double bond;
m represents an integer of 0 to 5;
n represents an integer of 0 to 5;
p represents an integer of 0 to 2;
q represents an integer of 0 to 4;
when m is two or more, a plurality of R²s may be the same as or different from each other, and when two R²s represent a C1―3 alkyl group and are on the same carbon atom, the R²s together may form a C3-7 saturated carbon ring;
when n is two or more, a plurality of R³s may be the same as or different from each other; and
when q is two or more, a plurality of R⁴s may be the same as or different from each other], a salt thereof, a solvate thereof, an N-oxide thereof, or a prodrug thereof.

Furthermore, further Axl inhibitors are described in WO 2007/030680, WO2007/057399, WO 2007/070872, WO 2008/045978, WO 2008/080134, WO 2008/083356, WO 2008/128072, WO 2008/083353, WO 2008/083354, WO 2008/083367, WO 2008/083357, WO 2009/007390, WO 2009/024825, WO 2009/047514, WO 2009/053737, WO 2009/054864, WO 2009/127417, WO 2010/005876, WO 2010/005879, WO 2010/090764, WO 2010/128659, WO 2012/028332, WO 2012/135800, WO 2013/074633, WO 2013/115280, WO 2013/162061, WO 2014/091265, WO 2016/006706, or WO 2016/097918.

In the present disclosure, a halogen atom denotes fluorine, chlorine, bromine, and iodine.

In the present disclosure, the C1―8 alkyl group includes a linear or branched C1―8 alkyl group. Examples thereof include methyl, ethyl, propyl, butyl, pentyl, hexyl, heptyl, octyl, isopropyl, isobutyl, sec-butyl, tert-butyl, and isomers thereof.

In the present disclosure, the C1―4 alkyl group includes a linear or branched C1-4 alkyl group. Examples thereof include a methyl group, an ethyl group, a propyl group, a butyl group, an isopropyl group, an isobutyl group, a sec-butyl group, and a tert-butyl group.

In the present disclosure, the C1―3 alkyl group includes a linear or branched C1―3 alkyl group. Examples thereof include a methyl group, an ethyl group, a propyl group, and an isopropyl group.

In the present disclosure, the C1―4 haloalkyl group denotes, for example, a fluoromethyl group, a chloromethyl group, a bromomethyl group, an iodomethyl group, a difluoromethyl group, a trifluoromethyl group, a 1-fluoroethyl group, a 2-fluoroethyl group, a 2-chloroethyl group, a pentafluoroethyl group, a 1-fluoropropyl group, a 2-chloropropyl group, a 3-fluoropropyl group, a 3-chloropropyl group, a 4,4,4-trifluorobutyl group, and a 4-bromobutyl group.

In the present disclosure, the C2-8 alkenyl group denotes, for example, a vinyl group, a propenyl group, a butenyl group, a pentenyl group, a hexenyl group, a heptenyl group, and an octenyl group, and isomers thereof, and the like.

In the present disclosure, the C2-8 alkynyl group denotes, for example, an ethynyl group, a propynyl group, a butynyl group, a pentynyl group, a hexynyl group, a heptynyl group, an octynyl group, and isomers thereof.

In the present disclosure, examples of the C1-4 alkoxy group include a methoxy group, an ethoxy group, a propoxy group, an isopropoxy group, a butoxy group, an isobutoxy group, a sec-butoxy group, or a tert-butoxy group.

In the present disclosure, the C2-4 alkenyloxy group denotes, for example, vinyloxy, propenyloxy, butenyloxy, and isomers thereof, and the like.

In the present disclosure, the C2-4 alkynyloxy group denotes, for example, ethynyloxy, propynyloxy, butynyloxy, and isomers thereof, and the like.

In the present disclosure, the C3-7 carbon ring denotes a C3-7 monocyclic carbon ring, and a carbon ring which may be partially or completely saturated, and examples thereof include cyclopropane, cyclobutane, cyclopentane, cyclohexane, cycloheptane, cyclobutene, cyclopentene, cyclohexene, cycloheptene, cyclobutadiene, cyclopentadiene, cyclohexadiene, cycloheptadiene, or benzene ring.

In the present disclosure, the C5-7 carbon ring denotes a C5-7 monocyclic carbon ring, and a carbon ring which may be partially or completely saturated, and examples thereof include cyclopentane, cyclohexane, cycloheptane, cyclopentene, cyclohexene, cycloheptene, cyclopentadiene, cyclohexadiene, cycloheptadiene, or benzene ring.

In the present disclosure, examples of the saturated C3-7 carbon ring include cyclopropane, cyclobutane, cyclopentane, cyclohexane, and cycloheptane.

In the present disclosure, the 4- to 7-membered heterocycle denotes 4- to 7-membered monocyclic heterocycle, which includes one to five heteroatoms selected from an oxygen atom, a nitrogen atom and a sulfur atom, and a part or all of which is saturated. Examples thereof include azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxetane, dihydrofuran, tetrahydrofuran, dihydropyran, tetrahydropyran, dihydrooxepine, tetrahydrooxepine, perhydrooxepine, thietane, dihydrothiophene, tetrahydrothiophene, dihydrothiopyran, tetrahydrothiopyran, dihydrothiepin, tetrahydrothiepin, perhydrothiepin, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole, (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepin, tetrahydrothiadiazepin, perhydrothiadiazepin, morpholine, thiomorpholine, oxathiane, dioxolane, dioxane, dithiolane, dithiane, pyrrole, imidazole, triazole, tetrazole, pyrazole, pyridine, pyrazine, pyrimidine, pyridazine, azepine, diazepine, furan, pyran, oxepin, thiophene, thiopyran, thiepin, oxazole, isoxazole, thiazole, isothiazole, furazan, oxadiazole, oxazine, oxadiazine, oxazepine, oxadiazepine, thiadiazole, thiazine, thiadiazine, thiazepine, or thiadiazepin ring.

In the present disclosure, the 5- to 7-membered cyclic group denotes a C5-7 carbon ring and a 5- to 7-membered heterocycle. Herein, the C5-7 carbon ring denotes the same meaning mentioned above, and the 5- to 7-membered heterocycle includes 5- to 7-membered unsaturated heterocycle and 5- to 7-membered saturated heterocycle. Examples of the 5- to 7-membered heterocycle include pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrooxepine, tetrahydrooxepine, perhydrooxepine, dihydrothiophene, tetrahydrothiophene, dihydrothiopyran, tetrahydrothiopyran, dihydrothiepin, tetrahydrothiepin, perhydrothiepin, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole, (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepin, tetrahydrothiadiazepin, perhydrothiadiazepin, morpholine, thiomorpholine, oxathiane, dioxolane, dioxane, dithiolane, dithiane, pyrrole, imidazole, triazole, tetrazole, pyrazole, pyridine, pyrazine, pyrimidine, pyridazine, azepine, diazepine, furan, pyran, oxepin, thiophene, thiopyran, thiepin, oxazole, isoxazole, thiazole, isothiazole, furazan, oxadiazole, oxazine, oxadiazine, oxazepine, oxadiazepine, thiadiazole, thiazine, thiadiazine, thiazepine, or thiadiazepin ring.

In the present disclosure, the 6-membered cyclic group denotes a C6 carbon ring and a 6-membered heterocycle. Examples thereof include cyclohexane, cyclohexene, cyclohexadiene, benzene, pyridine, pyrazine, pyrimidine, pyridazine, pyran, thiopyran, oxazine, oxadiazine, thiazine, thiadiazine, dihydropyridine, tetrahydro pyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydropyran, tetrahydropyran, dihydrothiopyran, tetrahydrothiopyran, dihydrooxazine, tetrahydrooxazine, dihydrooxadiazine, tetrahydrooxadiazine, dihydrothiazine, tetrahydrothiazine, dihydrothiadiazine, tetrahydrothiadiazine, morpholine, thiomorpholine, oxathiane, dioxane, and dithiane ring.

In the present disclosure, "when the C1-8 alkyl group represented by R¹ is a branched alkyl group, C1―3 alkyl groups branched from the same carbon atom may together form a saturated C3-7 carbon ring" denotes that in a partial structure of the following general formula (I): 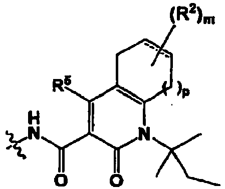 (wherein in the formula, all of the symbols have the same meanings as defined above), for example, when R¹ is a branched alkyl chain as represented in the above-mentioned formula, the alkyl chain branched from the same carbon atom, together with the carbon atom bonded thereto, forms a saturated carbon ring, as shown in the following formula: 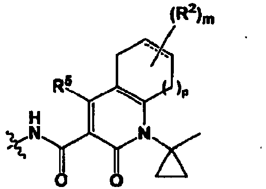 (wherein in the formula, all of the symbols have the same meanings as defined above).

In the present disclosure, "when two R²s represent a Cl-3 alkyl group and are on the same carbon atom, the R²s together with carbon atoms bonded thereto form a C3-7 saturated carbon ring" denotes that in a partial structure of the following general formula (I): 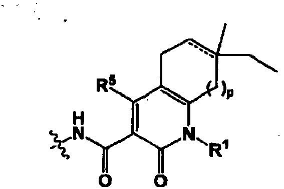 (wherein in the formula, all of the symbols have the same meanings as defined above), for example, when the R²s are an alkyl group and on the same carbon, the R²s together with the carbon atoms bonded thereto form a saturated carbon ring as shown in the following formula: 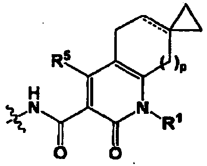 (wherein in the formula, all of the symbols have the same meanings as defined above).

In the present disclosure, "when two R²⁻¹'s represent a C1―3 alkyl group and are on the same carbon atom, the R²⁻¹'s together with the carbon atoms bonded thereto may form a C3-7 saturated carbon ring" means the same meaning as R² in the above-mentioned "when two R²s represent a C1―3 alkyl group, and are on the same carbon atom, the R²s together with the carbon atoms bonded thereto may form a C3-7 saturated carbon ring".

In the present disclosure, when m is one or more, one of the R²s is preferably an oxo group.

In the present disclosure, A is preferably CH.

In the present disclosure, R⁴ is preferably a C1―4 alkoxy group or an -OR⁴¹ group.

In the present disclosure, L is preferably -O-, -NH-, or -C(O)-.

In the present disclosure, ring1 is preferably a 6-membered cyclic group, and more preferably benzene or pyridine.

The Axl inhibitor to be used for the combination of the present disclosure is a compound represented by the general formula (I-1): 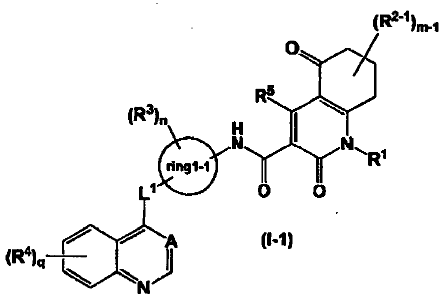 (wherein in the formula, R²⁻¹ represents (1) a C1-4 alkyl group, (2) a halogen atom, (3) a C1―4 haloalkyl group, or (4) an -OR²¹ group; m-1 represents an integer of 0 to 4; L¹ represents (1) -O-, (2) -NH-, or (3) -C(O)- ; ring1-1 represents benzene or pyridine; when m-1 is two or more, a plurality of R²⁻¹s may be the same as or different from each other; and when the two R²⁻¹s represent a C1―3 alkyl group and are on the same carbon atom, the R²⁻¹s together may form a C3-7 saturated carbon ring, and the other symbols have the same meanings as defined above), a salt thereof, a solvate thereof, an N-oxide thereof, or a prodrug thereof.

In the present disclosure, two bondings in the ring1 and ring1-1 are preferably bonded to the para position.

In the present disclosure, in the general formula (1-1), A is preferably CH, and R⁴ is preferably a C1―4 alkoxy group or an -OR⁴¹ group.

The Axl inhibitor to be used for the combination is more preferably a compound described in Example of WO2015/012298, and a pharmaceutically acceptable salt thereof or a hydrate thereof, and further more preferably (1) N-{5-[(6,7-dimethoxy-4-quinolinyl)oxy]-2-pyridinyl}-2,5-dioxo-1-phenyl-1,2,5,6,7,8-hexahydro-3-quinolinecarboxamide, (2) N-{5-[(6,7-dimethoxy-4-quinolinyl)oxy]-2-pyridinyl}-7,7-dimethyl -2,5-dioxo-1-phenyl-1,2,5,6,7,8-hexahydro-3-quinolinecarboxamide, (3) N-{5-[(6,7-dimethoxy-4-quinolinyl)oxy]-2-pyridinyl}-1-(2,2-dimethylpropyl)-2,5-dioxo-1,2,5,6,7,8-hexahydro-3-quinolinecarboxamide, (4) N-[5-({7-[3-(4-morpholinyl)propoxy]-4-quinolinyl}oxy)-2-pyridinyl]2,5-dioxo-1-phenyl-1,2,5,6,7,8-hexahydro-3-quinolinecarboxamide, (5) N-{4-[(6,7-dimethoxy-4-quinolinyl)oxy]-3-fluorophenyl}-2,5-dioxo-1-phenyl-1,2,5,6,7,8-hexahydro-3-quinolinecarboxamide, (6) N-{4-[(6,7-dimethoxy-4-quinolinyl)oxy] phenyl}-2,5-dioxo-1-phenyl-1,2,5,6,7,8-hexahydro-3-quinolinecarboxamide, (7) N- {5-[(6,7-dimethoxy-4-quinolinyl)oxy]-2-pyridinyl}-1-(4-fluorophenyl)-2,5-dioxo-1,2,5,6,7 ,8-hexahydro-3-quinolinecarboxamide, (8) N-{5-[(6,7-dimethoxy-4-quinolinyl)oxy]-2-pyridmyl}-1-(3-fluorophenyl)-2,5-dioxo-1,2,5,6,7,8-hexahydro-3-quinolinecarboxamide, (9) N-{5-[(6,7-dimethoxy-4-quinolinyl)oxy]-2-pyridinyl}-1-(2-fluorophenyl)-2,5-dioxo-1,2,5,6,7,8-hexahydro-3-quinolinecarboxamide, (10) N-{5-[(6,7-dimethoxy-4-quinazolinyl)oxy]-2-pyridinyl}-2,5-dioxo-1-phenyl-1,2,5,6,7,8-hexahydro-3-quinolinecarboxamide, (11) N-{5-[(6,7-dimethoxy-4-quinazolmyl)oxy]-2-pyridmyl}-1-(4-fluorophenyl)-2,5-dioxo-1,2,5,6,7,8-hexahydro-3-quinolinecarboxamide, (12) N- {5-[(6,7-dimethoxy-4-quinolmyl)oxy]-2-pyridinyl}-1-[(2S)-1-hydroxy-3-methyl-2-butanyl]-2,5-dioxo-1,2,5,6,7,8-hexahydro-3-quinolinecarboxamide, (13) N- {4-[(6,7-dimethoxy-4-quinolinyl)oxy]-3-fluorophenyl}-1-(3-fluorophenyl)-2,5-dioxo-1,2,5,6,7,8-hexahydro-3-quinolinecarboxamide, (14) N- {5-[(6,7-dimethoxy-4-quinolinyl)oxy]-2-pyridinyl}-6,6-dimethyl-2,5-dioxo-1-phenyl-1,2,5,6,7,8-hexahydro-3-quinolinecarboxamide, (15) N-[5-({6-methoxy-7-[3-(4-morpholinyl)propoxy]-4-quinolinyl}oxy)-2-pyridinyl]-2,5-dioxo-1-phenyl-1,2,5,6,7,8-hexahydro-3-quinolinecarboxamide, (16) N-(5-{[7-(3-hydroxy-3-methylbutoxy)-6-methoxy-4-quinolinyl]oxy}-2-pyridinyl)-2,5-dioxo-1-phenyl-1,2,5,6,7,8-hexahydro-3-quinolinecarboxamide, or (17) N-[5-({6-methoxy-7-[3-(1-pyrrolidinyl)propoxy]-4-quinolinyl}oxy)-2-pyridinyl]-2,5-dioxo-1-phenyl-1,2,5,6,7,8-hexahydro-3-quinolinecarboxamide, a pharmaceutically acceptable salt thereof or a hydrate thereof.

Particularly preferred is N-{5-[(6,7-dimethoxy-4-quinolinyl)oxy]-2-pyridinyl}-2,5-dioxo-1-phenyl-1,2,5,6,7,8-hexahydro-3-quinolinecarboxamide (hereinafter, also abbreviated as a "compound A") represented by the following structural formula: 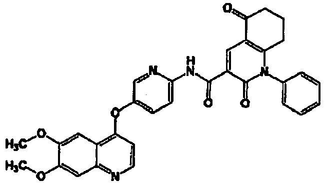 , a pharmaceutically acceptable salt thereof or a hydrate thereof.

In the present invention, unless specifically directed, all isomers are included. For example, an alkyl group includes linear and branched chain groups. In addition, geometrical isomers of double bonds, rings, and fused rings (E-, Z-, cis-, trans-isomers), optical isomers by the presence of an asymmetric carbon atom (R-, S-isomer, α-, β-configurations, enantiomers, diastereomers), optically active substances having optical activity (D-, L-, d-, 1-isomers), polar isomers according to chromatographic separation (more polar isomer, less polar isomer), equilibrium compound, rotamers, mixtures thereof at any rate, and racemic mixtures are all included in the present invention. Furthermore, the present invention also encompasses all isomers by tautomers.

Furthermore, the optical isomers of the present invention is not only limited to 100% pure isomers, but also may include other optical isomers with purity of less than 50%.

In the present invention, unless otherwise noted, as apparent to a person skilled in the art, the symbol:
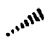 represents binding toward the back side of the plane of the paper (that is to say, the α-configuration),
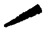 represents binding toward the front side of the plane of the paper (that is to say, the β-configuration), and
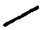 represents α-configuration, β-configuration or any mixture thereof.

The compound represented by the general formula (I) is converted into a corresponding salt by well-known methods. A salt is preferably a pharmaceutically acceptable salt and a water-soluble salt. Examples of a suitable salt include salts of an alkali metal (potassium, sodium, and the like), salts of an alkaline earth metal (calcium, magnesium, and the like), ammonium salts, or salts of a pharmaceutically acceptable organic amine (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine, and the like), acid addition salts (inorganic acid salts (hydrochloride, hydrobromide, hydroiodide, sulfate, phosphate, nitrate, and the like), organic acid salts (acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate, and the like).

The compound represented by the general formula (I) and a salt thereof can be also converted into a solvate. It is preferable that the solvate is low-toxic and water-soluble. Examples of a suitable solvate include solvates with water or with an alcoholic solvent (for example, ethanol). The solvate is preferably a hydrate.

The N-oxide of the compound represented by the general formula (I) denotes compounds represented by the general formula (I) in which a nitrogen atom is oxidized. Furthermore, the N-oxide of the compound represented by the general formula (I) may be salts of alkali (earth) metal salt, ammonium salt, organic amine salt, and acid addition salt mentioned above.

The prodrug of the compound represented by the general formula (I) denotes a compound which is converted into the compound represented by the general formula (I) by a reaction with an enzyme, stomach acid, and the like, in a living body. The prodrugs of the compound represented by the general formula (I) include: compounds in which the hydroxyl group is acylated, alkylated, phosphorylated, or borated (for example, the compounds in which the hydroxyl group is acetylated, palmitoylated, propanoylated, pivaloylated, succinylated, fumarylated, alanylated, or dimethylaminomethylcarbonylated) in cases where the compound has a hydroxyl group; and compounds wherein the carboxyl group of the compound represented by the general formula (I) is esterified or amidated (for example, compounds represented by the general formula (I) in which the carboxyl group is made into ethyl ester, isopropyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonyl ethyl ester, methylamide, and the like). These compounds can be produced by well-known methods. Furthermore, the prodrug of the compound represented by the general formula (I) may be hydrate or non-hydrate. Furthermore, the prodrug of the compound represented by the general formula (I) may be a compound which changes into the compound represented by the general formula (I) under physiological conditions, as described in "Development of Medicaments", vol.7 "Molecular Design", p.163-198, published by Hirokawa Shoten in 1990. In addition, the compound represented by the general formula (I) may be labeled with an isotope thereof (for example, ²H, ³H, ¹¹C, ¹³C, ¹⁴C, ¹³N, ¹⁵N, ¹⁵O, ¹⁷O, ¹⁸O, ³⁵S, ¹⁸F, ³⁶Cl, ¹²³I, ¹²⁵I, and the like).

The compound represented by the general formula (I) can be produced according to the method described in WO2015/012298.

The Axl inhibitor to be used for the combination of the present invention is usually administered systemically or locally, by oral or parenteral administration. Examples of oral agents include liquid drugs for internal use (for example, elixirs, syrups, pharmaceutically acceptable water-based agents, suspensions, and emulsions), and solid drugs for internal use (for example, tablets (including sublingual tablets and orally disintegrating tablets), pills, capsules (including hard capsules, soft capsules, gelatin capsules, and microcapsules), powders, granules, and lozenges). Examples of parenteral agents include liquid drugs (for example, injections (subcutaneous injections, intravenous injections, intramuscular injections, intraperitoneal injections, and drip agents), eye drops (for example, aqueous eye drops (aqueous eye lotions, aqueous eye lotion suspensions, viscous eye lotions, and solubilized eye lotions, etc.), and nonaqueous eye drops (for example, nonaqueous eye lotions and nonaqueous eye lotion suspensions), and the like), agents for external use (for example, ointments (ophthalmic ointments, and the like)), and ear-drops, and the like. These formulations may be controlled release agents such as rapid release formulations, sustained release formulations, and the like. These formulations can be produced by well-known methods, such as the methods described in The Japanese Pharmacopoeia.

Liquid drugs for internal use as oral agents can be produced by, for example, dissolving, suspending, or emulsifying an active component in a generally used diluent (for example, purified water, ethanol, mixture liquid thereof, or the like). A liquid drug may include a wetting agent, a suspension agent, an emulsifying agent, a sweetening agent, a flavoring material, an aromatic substance, a preservative, a buffer agent, and the like.

Solid drugs for internal use as the oral agent are formulated by, for example, mixing the active component with a vehicle (for example, lactose, mannitol, glucose, microcrystalline cellulose, and starch), a binder (for example, hydroxypropyl cellulose, polyvinylpyrrolidone, and magnesium metasilicate aluminate), a disintegrant (for example, calcium carboxymethylcellulose), a lubricant (for example, magnesium stearate), a stabilizer, a dissolution adjuvant (glutamic acid, aspartic acid, or the like), and the like, and formulating according to standard methods. As necessary, coating may be carried out with a coating agent (for example, saccharose, gelatin, hydroxypropyl cellulose, and hydroxypropyl methyl cellulose phthalate), and coating of two or more layers may be employed.

Agents for external use as parenteral agents are produced by well-known methods or generally used prescriptions. For example, an ointment may be produced by incorporation or melting of an active component into base material. The ointment base material is selected from well-known materials or generally used materials. For example, a single material or a mixture of two or more of materials are selected from higher fatty acids and higher fatty acid esters (for example, adipic acid, myristic acid, palmitic acid, stearic acid, oleic acid, adipate esters, myristate esters, palmitate esters, stearate esters, and oleate esters), waxes (for example, beeswax, spermaceti, and ceresin), surfactants (for example, polyoxyethylene alkyl ether phosphate esters), higher alcohols (for example, cetanol, stearyl alcohol, and cetostearyl alcohol), silicone oils (for example, dimethylpolysiloxane), hydrocarbons (for example, hydrophilic petrolatum, white petrolatum, purified lanolin, and liquid paraffin), glycols (for example, ethylene glycol, diethylene glycol, propylene glycol, polyethylene glycol, and macrogol), vegetable oils (for example, castor oil, olive oil, sesame oil, and turpentine oil), animal oils (for example, mink oil, egg yolk oil, squalane, and squalene), water, absorption promoters, and anti-irritants. Furthermore, a humectant, preservative, stabilizer, antioxidant, fragrance, and the like, may be included.

The injection agents as parenteral agents include solutions, suspensions, emulsions and solid injection agents to be dissolved or suspended in a solvent upon use. The injection agent is used by, for example, dissolving, suspending or emulsifying an active component in a solvent. Examples of the solvent include distilled water for injection, physiological saline, vegetable oils, alcohols such as propylene glycol, polyethylene glycol, ethanol, and mixtures thereof. Furthermore, the injection agent may contain a stabilizer, a dissolution aid (for example, glutamic acid, aspartic acid, and Polysorbate 80 (registered trademark), etc.), a suspending agent, an emulsifying agent, a soothing agent, a buffer, a preservative, and the like. Such an injection agent is produced by sterilizing at the final step or employing an aseptic process. Furthermore, it is also possible to produce an aseptic solid product, such as a freeze-dried product, and use the product following sterilization or dissolving the product in aseptic distilled water for injection or other solvents prior to use.

The dose of the Axl inhibitor to be used for the combination of the present invention is different depending on ages, body weights, symptoms, therapeutic effects, administration method, treatment time, and the like. The dose per adult is generally from 1 ng to 1000 mg per dose, once or several times per day by oral administration, or from 0.1 ng to 100 mg per dose, once or several times per day by parenteral administration, or continuous administration for 1 hour to 24 hours per day intravenously. Needless to say, as mentioned above, the dose to be used varies dependent on various conditions. Therefore, dose lower than the ranges specified above may be sufficient in some cases, and dose higher than the ranges specified above are needed in some cases.

When, for example, the compound A is used, a dose in one embodiment is 2 mg/kg to 20 mg/kg body weight, and preferably 2 mg/kg to 20 mg/kg body weight per day.

### (2) Immune checkpoint inhibitor

In the present invention, the immune checkpoint molecule means a molecule that exhibits an immunosuppression function by delivering an inhibitory co-signal. Examples of known immune checkpoint molecules include CTLA-4, PD-1, PD-L1 (programmed cell death-ligand 1), PD-L2 (programmed cell death-ligand 2), LAG-3 (Lymphocyte activation gene 3), TIM3 (T cell immunoglobulin and mucin-3), BTLA (B and T lympho-cyte attenuator), B7H3, B7H4, 2B4, CD 160, A2aR (adenosine A2a receptor), KIR (killer inhibitory receptor), VISTA(V-domain Ig-containing suppressor of T cell activation), TIGIT (T cell immunoglobulin and ITIM domain), and the like (see, Nature Reviews Cancer, 12, pp. 252-264, 2012, Cancer Cell, 27, pp. 450-461, 2015), but the immune checkpoint molecule is not particularly limited as long as it works according to the definition.

The immune checkpoint inhibitor used in the combination of the present invention is a substance that inhibits the function of the immune checkpoint molecule. The immune checkpoint inhibitor is not particularly limited as long as it can inhibit the function (signal) of the immune checkpoint molecule.

The immune checkpoint inhibitor is preferably an inhibitor of human immune checkpoint molecule, and further preferably a neutralized antibody against the human immune checkpoint molecule.

Examples of the immune checkpoint inhibitor include an inhibitor against the immune checkpoint molecule selected from the group consisting of CTLA-4, PD-1, PD-L1, PD-L2, LAG-3, TIM3, BTLA, B7H3, B7H4, 2B4, CD160, A2aR, KIR, VISTA, and TIGIT. The followings are examples of the immune checkpoint inhibitors.

Examples of immune checkpoint inhibitors include an anti-CTLA-4 antibody (for example, Ipilimumab (YERVOY (registered trademark)), Tremelimumab, AGEN-1884), anti-PD-1 antibody (for example, nivolumab (OPDIVO (registered trademark)), REGN-2810, Pembrolizumab (KEYTRUDA (registered trademark)), PDR-001, BGB-A317, AMP-514 (MEDI0680), BCD-100, IBI-308, JS-001, PF-06801591, and TSR-042), an anti-PD-Ll antibody (for example, Atezolizumab (RG7446 and MPDL3280A), Avelumab (PF-06834635 and MSB0010718C), Durvalumab (MEDI4736), BMS-936559, CA-170, and LY-3300054), anti-PD-L2 antibody (for example, rHIgM12B7), PD-L1 fusion protein, PD-L2 fusion protein (for example, AMP-224), an anti-Tim-3 antibody (for example, MBG453), an anti-LAG-3 antibody (for example, BMS-986016, and LAG525), and an anti-KIR antibody (for example, Lirilumab). Furthermore, antibodies including heavy chain and light chain complementarity determining regions (CDRs) or variable region (VR) of the above-mentioned known antibodies are also one embodiment of the immune checkpoint inhibitor. Examples of further embodiments of the anti-PD-1 antibody include an antibody including heavy chain and light chain complementarity determining regions (CDRs) or variable region (VR) of nivolumab.

Examples of antibodies including the heavy chain and light chain complementarity determining regions (CDRs) or variable region (VR) of nivolumab include (1) an anti-PD-1 antibody including (a) a heavy chain variable region CDR1 including an amino acid sequence set forth in SEQ ID No. 3, (b) a heavy chain variable region CDR2 including an amino acid sequence set forth in SEQ ID No. 4, (c) a heavy chain variable region CDR3 including an amino acid sequence set forth in SEQ ID No. 5, (d) a light chain variable region CDR1 including an amino acid sequence set forth in SEQ ID No. 6, (e) a light chain variable region CDR2 including an amino acid sequence set forth in SEQ ID No. 7, and (f) a light chain variable region CDR3 including an amino acid sequence set forth in SEQ ID No. 8, or (2) an anti-PD-1 antibody including a heavy chain variable region including an amino acid sequence set forth in SEQ ID No. 1 and a light chain variable region including an amino acid sequence set forth in SEQ ID No. 2 (preferably, an isolated human monoclonal IgG4 antibody of (1) or (2)).

The immune checkpoint inhibitor used in the combination of the present disclosure is an anti-CTLA-4 antibody, an anti-PD-1 antibody, an anti-PD-Ll antibody, an anti-PD-L2 antibody, PD-L1 fusion protein, and PD-L2 fusion protein; more preferably an anti-PD-1 antibody, anti-PD-Ll antibody, anti-PD-L2 antibody, PD-L1 fusion protein, and PD-L2 fusion protein; the immune checkpoint inhibitor used in the combination of the present invention is an anti-PD-1 antibody. The anti-PD-1 antibody is preferably an antibody (including nivolumab) including heavy chain and light chain complementarity determining regions (CDRs) or variable region (VR) including nivolumab, and further preferably nivolumab.

Any one of or a plurality of types of antibodies or fusion protein among these immune checkpoint inhibitors can be used in combination with the Axl inhibitor according to the present disclosure.

The dose of the immune checkpoint inhibitor to be used for the combination of the present invention is different depending on ages, body weights, symptoms, therapeutic effects, administration methods, treatment time, and the like, but is adjusted such that the optimum desired effect can be exhibited.

For example, when the anti-PD-1 antibody is used, the dose in one embodiment is 0.1 to 20 mg/kg body weight. Furthermore, when the antibody (for example, nivolumab) including a heavy chain and light chain complementarity determining regions (CDRs) or variable region (VR) of nivolumab is used, the dose in one embodiment is 0.3 to 10 mg/kg body weight, and preferably 2 mg/kg or 3 mg/kg body weight.

### [Toxicity]

The combination of the present invention has sufficiently low toxicity, and, therefore, it can be used safely as pharmaceutical preparations.

### [Application to pharmaceutical preparations]

In one embodiment, diseases to be treated by the combination of the present invention include cancer. The cancer is not particularly limited, and examples thereof include leukemia (for example, acute myeloid leukemia, chronic myeloid leukemia, acute lymphatic leukemia, and chronic lymphatic leukemia), malignant lymphoma (Hodgkin lymphoma and non-Hodgkin lymphoma (for example, adult T-cell leukemia, follicular lymphoma, and diffuse large B-cell lymphoma)), multiple myeloma, osteomyelodysplasia syndrome, head and neck cancer, esophageal cancer, esophageal adenocarcinoma, gastric cancer, colon cancer, colorectal cancer, rectum cancer, liver cancer (for example, hepatocellular cancer), gallbladder cancer and bile duct cancer, biliary tract cancer, pancreatic cancer, thyroid cancer, lung cancer (for example, non-small cell lung cancer (for example, squamous epithelium non-small cell lung cancer, non-squamous epithelium non-small cell lung cancer), and small-cell lung cancer), breast cancer, ovarian cancer (for example, serous ovarian cancer), cervical cancer, uterine body cancer, endometrial cancer, vaginal cancer, vulvar cancer, renal cancer (for example, renal cell carcinoma), urothelial carcinoma (for example, urinary bladder cancer, and upper urinary tract cancer), prostate cancer, testicular tumor (for example, germ cell cancer), bone and soft tissue sarcoma, skin cancer (for example, uveal malignant melanoma, malignant melanoma, and Merkel cell cancer), glioma, brain tumor (for example, glioblastoma), pleural mesothelioma, and unknown primary cancer). Among them, for example, with regards to patients who have not obtained sufficient therapeutic effect by a single administration of the immune checkpoint inhibitor or the Axl inhibitor, it is expected that the combination of the present invention can exhibit the maximum anti-tumor effect. Furthermore, the combination of the present invention can reduce the dose of each of the pharmaceutical drugs. As a result, reduction of adverse reactions can be expected.

In one embodiment, the combination of the present invention can be applied to treatment of metastatic carcinoma or suppression of metastasis.

In one embodiment, the combination of the present invention suppresses recurrence.

In the present invention, treatment means bringing about at least one effect among reduction of the size of a tumor, suppression of tumor growth (retardation or stopping), suppression of tumor metastasis (retardation or stopping), suppression of the recurrence (prevention or retardation), and alleviation of one or a plurality of symptoms associated with cancer.

In one embodiment, the combination of the present invention can be used for treatment of colon cancer or pancreatic cancer.

In the present invention, combined administration includes simultaneous administration of compounds having the same or different dosage form, or separate administration of compounds (for example, sequential administration). The "administering in combination" in the present invention has the same meaning as combined administration, and, more specifically, may include administering in the form of a combination drug with these components blended in one formulation, or administering as separate formulations. Administration as separate formulations includes simultaneous administration and administration at different times. With regards to the administration at different times, the compound A may be administered before other drugs, or alternatively, the other drugs may be administered before the compound A. The method for the administration of these drugs may be the same as or different from each other.

In the present invention, the combination of the present invention may be administered in combination with other drugs (for example, well-known anti-cancer drugs) for the purposes of (1) supplementing and/or enhancing therapeutic effect, (2) improving the kinetics, improving absorption, and reducing the dose, and/or (3) eliminating adverse reactions of the compound.

### [EXAMPLES]

Hereinafter, the present invention is described specifically with reference to Examples.

As the Axl inhibitor represented by the general formula (I), N-{5-[(6,7-dimethoxy-4-quinolinyl)oxy]-2-pyridinyl}-2,5-dioxo-1-phenyl-1,2,5,6,7,8-hexahydro-3-quinolinecarboxamide (a compound A) was used. The compound A can be manufactured by well-known methods, such as the method described in Example 5 of WO 2015/012298.

Biological Example 1: Evaluation of anti-tumor action by combined use of compound A and anti-PD-1 antibody on mouse colon cancer cell line MC38 subcutaneous cancer-bearing model (in vivo)

For the compound A, mouse feed CRF-1 containing 0.0013% or 0.013% compound A was manufactured by Oriental Yeast Co., Ltd. so as to be administered at a dose of 2 or 20 mg/kg/day via diet. 4H2 as the anti-mouse PD-1 antibody, which had been prepared in KITAYAMA LABES CO., LTD., was obtained. C57/BL6 mouse colon cancer cell line MC38 was subcutaneously transplanted into dorsal parts of allogenic and syngeneic mice (C57/BL6, female, 6 weeks old (Charles River Laboratories Japan, Inc.)) to produce MC38 subcutaneous cancer-bearing mice. On the day when the average of tumor volumes reached 150 mm³, group assignment was carried out based on the tumor volumes (the day on which administration was started was defined as Day 0). To the MC38 subcutaneous cancer-bearing mice, PBS (on days 0, 6, 12, 18, and 24, intraperitoneal administration, n=9), 4H2 (on Day 0, 20 mg/kg/day and on days 6, 12, 18, and 24, 10 mg/kg/day, intraperitoneal administration, n=8), the compound A (on days 0 to 28, 2 mg/kg, administration via diet (n=9)), the compound A (on days 0 to 28, 20 mg/kg, administration via diet (n=9)), combined use of 4H2 and the compound A (compound A, 2 mg/kg (n=9)), and combined use of 4H2 and the compound A (compound A, 20 mg/kg (n=9)) were administered. Change of the volume of tumor was observed continuously until the 28th day. Note here that, from the viewpoint of the animal ethics, a mouse whose tumor exceeded 3000 mm³ in volume was euthanatized, and evaluation for the group to which the euthanatized mouse belonged was stopped at this point. The results are shown in FIG. 1 and Table 1.

The average volume of tumors on day 14 was 1826.2 mm³ in the case of PBS, 860.9 mm³ in the case of 4H2, 1379.5 mm³ in the case of the compound A (2 mg/kg), and 639.3 mm³ in the case of the compound A (20 mg/kg). Meanwhile, the average volume of tumors with regards to combined uses was 216.0 mm³ in the case of the combined use of 4H2 and the compound A (2 mg/kg), and 145.3 mm³ in the case of the combined use of 4H2 and the compound A (20 mg/kg). The combined uses showed stronger effects as compared with single administrations of each pharmaceutical drug. Furthermore, while cancer had been eliminated only in one mouse each in the cases of single administrations of 4H2 or the compound A in the respective doses, in the case of the combined use of 4H2 and the compound A (2 mg/kg) and the case of the combined use of 4H2 and the compound A (20 mg/kg), cancer had been eliminated in three and six mice, respectively.

According to the above, it was verified that the combined use of the compound A with the anti-PD-1 antibody exhibited a strong anti-tumor effect.

**[Table 1]**

| | Number of cases where tumor was eliminated |
|---|---|
| PBS | 0/9 |
| 4H2 | 1/8 |
| Compound A (2 mg/kg) | 1/9 |
| Compound A (20 mg/kg) | 1/9 |
| 4H2 + Compound A (2 mg/kg) | 3/9 |
| 4H2 + Compound A (20 mg/kg) | 6/9 |

Biological Example 2: Evaluation of anti-tumor action by combined use of compound A and anti-PD-1 antibody on mouse pancreatic cancer cell line Pan02 orthotopic implantation model (in vivo)

For the compound A, mouse feed CRF-1 containing 0.0013% or 0.0040% compound A was manufactured by Oriental Yeast Co., Ltd. so as to be administered at the dose of 2 or 6 mg/kg/day via diet. 4H2 as the anti-mouse PD-1 antibody, which had been prepared in KITAYAMA LABES CO., LTD., was obtained. C57/BL6 mouse pancreatic cancer cell line Pan02 was transplanted into the pancreas of the allogenic and syngeneic mice (C57/BL6, female, 6 weeks old (Charles River Laboratories Japan, Inc.)) to produce Pan02 orthotopic implantation mice. Following 7 days after transplantation, based on the body weight, the mice were assigned to four groups, i.e., a vehicle group, a 4H2 group, a combined use group of 4H2 and the compound A (2 mg/kg/day), and a combined use group of 4H2 and the compound A (6 mg/kg/day) (each n=12). The day on which group assignment was carried out was defined as Day 0. PBS (10 mL/kg, every six days after Day 0) was intraperitoneally administered to the vehicle group, and 4H2 was intraperitoneally administered (20mg/10mL/kg on Day 0 and 10mg/10mL/kg every six day thereafter) to the 4H2 group, the combined use group of 4H2 and the compound A (2 mg/kg/day), and the combined use group of 4H2 and the compound A (6 mg/kg/day). Furthermore, from Day 0, CRF-1 containing 0.0013% compound A was fed to the combined use group of 4H2 and the compound A (2 mg/kg/day), and CRF-1 containing 0.0040% compound A was fed to the combined use group of 4H2 and the compound A (6 mg/kg/day), respectively. From Day 0 to Day 114, the survival time of each mouse in each group was evaluated. Note here that, from the viewpoint of the animal ethics, mice which showed any one of the conditions among not being able to take food and water due to paralysis of both hind limbs, showing no reaction to light stimulation given by fingers, having respiratory failure, crouching and not moving, showing loss of body weight by more than 20% within 2 to 3 days, and showing loss of body weight by more than 25% within 7 days were euthanatized, and the date was defined as their day of death. The results are shown in FIG. 2.

The median value of the survival time (from the day of group assignment to the day of death) of the 4H2 group was 52 days. The survival time was significantly extended as compared with 43 days of the vehicle group. The median value of the survival time of the combined use group of 4H2 and the compound A (6 mg/kg/day) was 64 days, and the survival time was significantly extended as compared with the 4H2 group.

According to the above, it was verified that the combined use of the compound A and the anti-PD-1 antibody exhibits a strong anti-tumor effect.

### [Industrial Applicability]

The combination of the present invention is useful for cancer therapy since it exhibits a strong anti-tumor effect.

### [Sequence Listing Free Text]

SEQ ID No. 1: nivolumab _VH
SEQ ID No. 2: nivolumab _VL
SEQ ID No. 3: nivolumab _VH CDR1
SEQ ID No. 4: nivolumab _VH CDR2
SEQ ID No. 5: nivolumab _VH CDR3
SEQ ID No. 6: nivolumab _VL CDR1
SEQ ID No. 7: nivolumab _VL CDR2
SEQ ID No. 8: nivolumab _VL CDR3

## Claims

1. A drug for use in cancer therapy, comprising a combination of an Axl inhibitor and an anti-PD-1 antibody, the Axl inhibitor being N-{5-[(6,7-dimethoxy-4-quinolinyl)oxy]-2-pyridinyl}-2,5-dioxo-1-phenyl-1,2,5,6,7,8-hexahydro-3-quinolinecarboxamide, a salt thereof, a solvate thereof, an N-oxide thereof, or a prodrug thereof.

2. The drug for use in cancer therapy according to claim 1, wherein the Axl inhibitor and the anti-PD-1 antibody are for simultaneous administration or separate administration.

3. The drug for use according claim 1, wherein the cancer is leukemia selected from acute myeloid leukemia, chronic myeloid leukemia, acute lymphatic leukemia, and chronic lymphatic leukemia, malignant lymphoma selected from Hodgkin lymphoma and non-Hodgkin lymphoma selected from adult T-cell leukemia, follicular lymphoma, and diffuse large B-cell lymphoma, multiple myeloma, osteomyelodysplasia syndrome, head and neck cancer, esophageal cancer, esophageal adenocarcinoma, gastric cancer, colon cancer, colorectal cancer, rectum cancer, liver cancer, gallbladder cancer and bile duct cancer, biliary tract cancer, pancreatic cancer, thyroid cancer, lung cancer selected from non-small cell lung cancer selected from squamous epithelium non-small cell lung cancer, non-squamous epithelium non-small cell lung cancer, and small-cell lung cancer, breast cancer, ovarian cancer, cervical cancer, uterine body cancer, endometrial cancer, vaginal cancer, vulvar cancer, renal cancer, urothelial carcinoma selected from urinary bladder cancer, and upper urinary tract cancer, prostate cancer, testicular tumor, bone and soft tissue sarcoma, skin cancer selected from uveal malignant melanoma, malignant melanoma, and Merkel cell cancer, glioma, brain tumor, pleural mesothelioma, and unknown primary cancer.

4. The drug for use according claim 1, wherein the cancer is colon cancer or pancreatic cancer.

5. A pharmaceutical composition for use in cancer therapy comprising a combination of an Axl inhibitor and an anti-PD-1 antibody as active components, the Axl inhibitor being N-{5-[(6,7-dimethoxy-4-quinolinyl)oxy]-2-pyridinyl}-2,5-dioxo-1-phenyl-1,2,5,6,7,8-hexahydro-3-quinolinecarboxamide, a salt thereof, a solvate thereof, an N-oxide thereof, or a prodrug thereof.

6. An Axl inhibitor being N-{5-[(6,7-dimethoxy-4-quinolinyl)oxy]-2-pyridinyl}-2,5-dioxo-1-phenyl-1,2,5,6,7,8-hexahydro-3-quinolinecarboxamide,
a salt thereof, a solvate thereof, an N-oxide thereof, or a prodrug thereof,
for use in the treatment of cancer,
wherein the Axl inhibitor is administered in combination with an anti-PD-1 antibody.

7. The drug for use according to any one of claims 1-4, the pharmaceutical composition for use according to claim 5 or the Axl inhibitor for use according to claim 6, wherein the anti-PD-1 antibody is nivolumab.

## Patentansprüche

1. Ein Arzneimittel zur Verwendung in der Krebstherapie, umfassend eine Kombination aus einem Axl-Inhibitor und einem Anti-PD-1-Antikörper, wobei der Axl-Inhibitor N-{5-[(6,7-Dimethoxy-4-chinolinyl)oxy]-2-pyridinyl}-2,5-dioxo-1-phenyl-1,2,5,6,7,8-hexahydro-3-chinolincarboxamid, ein Salz davon, ein Solvat davon, ein N-Oxid davon oder ein Prodrug davon ist.

2. Das Arzneimittel zur Verwendung in der Krebstherapie gemäß Anspruch 1, wobei der Axl-Inhibitor und der Anti-PD-1-Antikörper zur gleichzeitigen oder getrennten Verabreichung vorgesehen sind.

3. Das Arzneimittel zur Verwendung gemäß Anspruch 1, wobei der Krebs Leukämie, ausgewählt aus akuter myeloischer Leukämie, chronischer myeloischer Leukämie, akuter lymphatischer Leukämie und chronischer lymphatischer Leukämie, malignes Lymphom, ausgewählt aus Hodgkin-Lymphom und Non-Hodgkin-Lymphom, ausgewählt aus adulter T-Zell-Leukämie, follikulärem Lymphom, und diffus großzelligem B-Zell-Lymphom, multiplem Myelom, Osteomyelodysplasie-Syndrom, Kopf- und Halskrebs, Speiseröhrenkrebs, Speiseröhrenadenokarzinom, Magenkrebs, Dickdarmkrebs, kolorektalem Krebs, Rektumkrebs, Leberkrebs, Gallenblasenkrebs und Gallengangskrebs, Gallenwegskrebs, Pankreaskrebs, Schilddrüsenkrebs, Lungenkrebs, ausgewählt aus nicht-kleinzelligem Lungenkrebs, ausgewählt aus nicht-kleinzelligem Plattenepithel-Lungenkrebs, nicht-plattenepithelischem nicht-kleinzelligem Lungenkrebs und kleinzelligem Lungenkrebs, Brustkrebs, Eierstockkrebs, Gebärmutterhalskrebs, Gebärmutterkörperkrebs, Endometriumkrebs, Vaginalkrebs, Vulvakrebs, Nierenkrebs, Urothelkarzinom, ausgewählt aus Harnblasenkrebs und Krebs der oberen Harnwege, Prostatakrebs, Hodentumor, Knochen- und Weichteilsarkom, Hautkrebs, ausgewählt aus uvealem malignem Melanom, malignem Melanom und Merkelzellkrebs, Gliom, Hirntumor, Pleuramesotheliom und unbekanntem Primärkrebs.

4. Das Arzneimittel zur Verwendung gemäß Anspruch 1, wobei der Krebs Dickdarmkrebs oder Bauchspeicheldrüsenkrebs ist.

5. Eine pharmazeutische Zusammensetzung zur Verwendung in der Krebstherapie, umfassend eine Kombination eines Axl-Inhibitors und eines Anti-PD-1-Antikörpers als aktive Komponenten, wobei der Axl-Inhibitor N-{5-[(6,7-Dimethoxy-4-chinolinyl)oxy]-2-pyridinyl}-2,5-dioxo-1-phenyl-1,2,5,6,7,8-hexahydro-3-chinolincarboxamid, ein Salz davon, ein Solvat davon, ein N-Oxid davon oder ein Prodrug davon ist.

6. Ein Axl-Inhibitor, der N-{5-[(6,7-Dimethoxy-4-chinolinyl)oxy]-2-pyridinyl}-2,5-dioxo-1-phenyl-1,2,5,6,7,8-hexahydro-3-chinolincarboxamid ist,
ein Salz davon, ein Solvat davon, ein N-Oxid davon oder ein Prodrug davon,
zur Verwendung in der Krebsbehandlung,
wobei der Axl-Inhibitor in Kombination mit einem Anti-PD-1-Antikörper verabreicht wird.

7. Das Arzneimittel zur Verwendung gemäß einem beliebigen der Ansprüche 1-4, die pharmazeutische Zusammensetzung zur Verwendung gemäß Anspruch 5 oder der Axl-Inhibitor zur Verwendung gemäß Anspruch 6, wobei der Anti-PD-1-Antikörper Nivolumab ist.

## Revendications

1. Médicament pour l'utilisation en thérapie anticancéreuse, comprenant une combinaison d'un inhibiteur Axl et d'un anticorps anti-PD-1, l'inhibiteur Axl étant le N-{5-[(6,7-diméthoxy-4-quinolinyl)oxy]-2-pyridinyl}-2,5-dioxo-1-phényl-1,2,5,6,7,8-hexahydro-3-quinolinecarboxamide, un sel de celui-ci, un solvate de celui-ci, un N-oxyde de celui-ci, ou un promédicament de celui-ci.

2. Médicament pour l'utilisation en thérapie anticancéreuse selon la revendication 1, l'inhibiteur Axl et l'anticorps anti-PD-1 servant à l'administration simultanée ou à l'administration séparée.

3. Médicament pour l'utilisation selon la revendication 1, le cancer étant la leucémie sélectionnée parmi la leucémie myéloïde aiguë, la leucémie myéloïde chronique, la leucémie lymphatique aiguë, et la leucémie lymphatique chronique, le lymphome malin sélectionné parmi le lymphome de Hodgkin et le lymphome non hodgkinien sélectionné parmi la leucémie à cellules T chez l'adulte, le lymphome folliculaire, et le lymphome diffus à grandes cellules B, le myélome multiple, le syndrome d'ostéomyélodysplasie, le cancer de la tête et du cou, le cancer de l'œsophage, l'adénocarcinome de l'œsophage, le cancer de l'estomac, le cancer du côlon, le cancer colorectal, le cancer du rectum, le cancer du foie, le cancer de la vésicule biliaire et le cancer du conduit biliaire, le cancer du tractus biliaire, le cancer du pancréas, le cancer de la thyroïde, le cancer du poumon sélectionné parmi le cancer du poumon non à petites cellules sélectionné parmi le cancer du poumon non à petites cellules de l'épithélium squameux, le cancer du poumon non à petites cellules de l'épithélium non squameux, et le cancer du poumon à petites cellules, le cancer du sein, le cancer de l'ovaire, le cancer du col de l'utérus, le cancer du corps utérin, le cancer de l'endomètre, le cancer du vagin, le cancer de la vulve, le cancer du rein, le carcinome urothélial sélectionné parmi le cancer de la vessie, et le cancer du tractus urinaire supérieur, le cancer de la prostate, la tumeur du testicule, le sarcome des os et du tissu mou, le cancer de la peau sélectionné parmi le mélanome malin uvéal, le mélanome malin, et le cancer des cellules de Merkel, le gliome, la tumeur au cerveau, le mésothéliome pleural, et le cancer primaire inconnu.

4. Médicament pour l'utilisation selon la revendication 1, le cancer étant le cancer du côlon ou le cancer du pancréas.

5. Composition pharmaceutique pour l'utilisation en thérapie anticancéreuse comprenant une combinaison d'un inhibiteur Axl et d'un anticorps anti-PD-1 comme composants actifs, l'inhibiteur Axl étant le N-{5-[(6,7-diméthoxy-4-quinolinyl)oxy]-2-pyridinyl}-2,5-dioxo-1-phényl-1,2,5,6,7,8-hexahydro-3-quinolinecarboxamide, un sel de celui-ci, un solvate de celui-ci, un N-oxyde de celui-ci, ou un promédicament de celui-ci.

6. Inhibiteur Axl étant le N-{5-[(6,7-diméthoxy-4-quinolinyl)oxy]-2-pyridinyl}-2,5-dioxo-1-phényl-1,2,5,6,7,8-hexahydro-3-quinolinecarboxamide, un sel de celui-ci, un solvate de celui-ci, un N-oxyde de celui-ci, ou un promédicament de celui-ci, pour l'utilisation dans le traitement du cancer,
l'inhibiteur Axl étant administré en combinaison avec un anticorps anti-PD-1.

7. Médicament pour l'utilisation selon l'une quelconque des revendications 1 à 4, composition pharmaceutique pour l'utilisation selon la revendication 5 ou inhibiteur Axl pour l'utilisation selon la revendication 6, l'anticorps anti-PD-1 étant le nivolumab.
